# EUROPEAN PATENT SPECIFICATION

(11) **EP 2 515 848 B1**
(45) Date of publication and mention of the grant of the patent: **12.11.2014**
(21) Application number: 10773420.4
(22) Date of filing: 22.09.2010
(51) Int. Cl.: A61K 9/00, A61K 45/06, A61K 31/46, A61K 31/58, A61K 31/352, A61K 31/47, A61P 11/06, A61P 11/08

(54) **DRY POWDER COMBINATION OF TIOTROPIUM, A CORTICOSTEROID AND A CROMOGLICIC ACID DERIVATIVE**
TROCKENPULVERKOMBINATION AUS TIOTROPIUM, EINEM CORTICOSTEROID UND EINEM CROMOGLICINSÄUREDERIVAT
COMBINAISON DE POUDRE SÈCHE CONSTITUÉE DE TIOTROPIUM, D'UN CORTICOSTÉROÏDE ET D'UN DÉRIVÉ D'ACIDE CROMOGLICIQUE

(30) Priority: 25.12.2009 TR 200909792
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000189
(87) International publication number: WO 2011/078818

(56) References cited:
- WO-A2-02/36106
- WO-A2-2008/102128
- US-B1- 6 475 467

## Description

The present invention relates to a pharmaceutical composition that is used by inhalation route, containing tiotropium combined with corticosteroid, and effective amount of cromolyn derivatives used for the treatment of respiratory disorder by inhalation route.

Tiotropium (Formula I) is an anticholinergic agent with chemical name (1α, 2β, 4β, 7β)-7-[(hydroxidi-2-thienylacetyl)oxy]-9,9-dimethyl-3-oxa-9-azoniatricyclo [3.3.1.0^{2,4}] nonane.

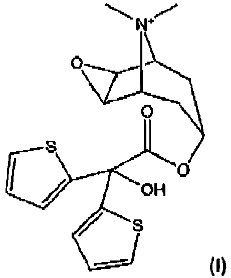

Tiotropium is described in European patent application EP0418716 for the first time. The patent relates to processes for preparing Tiotropium, pharmaceutical compositions containing tiotropium, long-acting, strong anticholinergic activity of tiotropium and use of it in the treatment of respiratory disorders.

Tiotropium is a long acting, strong anthichlolinergic bronchodilator used orally in the treatment of respiratory system diseases. Tiotropium is marketed under the trade name "Spiriva" as a dry powder form in capsules and used via inhalation device under the trade name "Handyhaler". Tiotropium is separated slowly from M₁ and M₃ receptors that cause broncho-construction, and it is separated rapidly from M₂ receptors that inhibit release of acetylcholine from cholinergic nerve endings. This situation that takes place in lung receptors demonstrates long acting bronchodilator activity of the drug.

An example of using Tiotropium with corticosteroid substances is disclosed in patent application US2004/0266869. In this application using combination of ciclesonide with tiotropium is especially mentioned and it is indicated that when using ciclesonide in combination with one or more anticholinergic agents, there is a decreasing in side effects, especially the ones caused by use of corticosteroids.

Another example of combined use of tiotropium with ciclesonide is given in document US 2004/0176338. The document is stating that an unexpected therapeutic effect is observed in treatment of inflammatory and obstructive diseases when one or more anticholinergics are used in combination with one or more anticholinergics and that this may reduce the unwanted side effects that occur when corticosteroids are administered. The application provides a dry powder formulation and gas containing aerosol formulations comprising a combination of tiotropium bromide and ciclesonide.

Inhalation route is a commonly preferred treatment method for respiratory disorders especially chronic disorders such as asthma and chronic obstructive pulmonary disorder (COPD) which threaten a large portion of the society. The reason is that drug reaches directly and rapidly to target area, accordingly lower doses of drug show desired effect by comparison with doses required for use via oral or parenteral route; and drug which is used in lower doses, show less side-effect than the drug administered via oral and parenteral route.

When inhaled medications reach the target area, they do not enter blood circulation. Thus, problems that change the activity of drug, such as; low solubility, low permeability, irritation, formation of unwanted metabolites and gastrointestinal problems, such as; decrease in bioavailability due to food are experienced in the lowest possible level.

Medicine formulations used in locally via inhalation can be used in dry powder inhaler, inhaler with pressured gases or nebulization devices. Although each of these different devices have positive and negative aspects, especially dry powder inhalers come into prominence with its ease of use. However, for dry powder formulations, there is still need to develop different transmission method to convey the effective doses to the lung.

Lactose, which is a disaccharide, is generally used as a carrier in dry powder formulations developed for use via inhalation. The amount of lactose used is usually in the range of 10 mg to 25 mg, which is approximately 500-2500 times more than the amount of active agent, for a single dose. Lactose is milligram in weight when the active agent is in microgram. However, a large amount of lactose used as a carrier in dry powder formulation caused side effects such as coughing, throat irritation, etc. Also, dry powder formulations containing large amount of lactose when used by patient with allergy and/or lactose intolerance (sensitivity to lactose taking high doses), causes symptoms such as nausea, stomach cramps, overfullness of the stomach, swelling of stomach, flatus, diarrhea, hives plaque. Then, 15 to 25% of each dose of the drugs administered by inhalation can reach the target area, the rest is mostly swallowed. Because of these drawbacks, dry powder formulations containing tiotropium and corticosteroid or pharmaceutically acceptable salts, need to be developed in terms of efficiency and safety.

Salts of cromoglicic acid and nedocromil are used for the treatment of bronchospasm in pharmacology. Because of this property, it is used for the treatment of asthma, allergic rhinitis, allergic and vernal conjunctivitis diseases.

Products which are marketed by the trademarks of "Intal" and "Cromohexal" provide 20 mg sodium cromoglicate per dose.

It is known that cromoglicic acid derivatives are used as a solubility enhancing agent in some aerosol formulations. For instance, US6475467 relates to use of a compound selected from cromoglicic acid and nedocromil derivatives in an ineffective amount in aerosol formulation that is in the form of suspension for increasing the dispersion of active agent in this suspension The present invention relates to use of salts of cromoglicic acid and/or nedocromil (B) in dry powder formulations containing tiotropium bromide (A) combined with ciclesonide or pharmaceutically acceptable salts thereof (S) administered by inhalation for the treatment of respiratory diseases. Initially, for the purpose of reducing the amount of lactose and alleviation of side effects caused by lactose, (B) has been added to the dry powder formulation which includes (A) and (S). But surprisingly, it has been found that the combination has a synergistic effect and provides an unexpected benefit for treatment of respiratory disease like asthma, chronic obstructive lung disease, when the amount of (B) was increased and used in an effective amount in the prepared dry powder formulation which include (A) and (S). Therefore, according to the present invention, when salts of cromoglicic acid and/or nedocromil (B) are used in dry powder formulations which include tiotropium bromide (A) combined with ciclesonide or pharmaceutically acceptable derivatives thereof (S), both required amount of carrier is reduced thus the side-effects caused by carrier are alleviated, and a synergistic effect is obtained for the treatment of respiratory diseases.

The present invention provides a medicament composition containing tiotropium bromide (A) with ciclesonide or a pharmaceutically acceptable derivative such as salt, solvate, hydrate, polymorph, enantiomer, racemate, amorph and/or crystal form thereof (S) as well as a salt of cromoglicic acid and/or nedocromil (B) in an effective amount, for use in the treatment of respiratory diseases.

The medicament composition in accordance with invention optionally contains a carrier in addition to the substances mentioned above.

In another aspect, the invention provides use of (A), (S) and (B) defined hereinbefore in effective amounts for the preparation of a medicament which is used for the treatment of respiratory diseases especially allergic diseases.

The inventors encountered a problem of inability to deliver an effective amount of dose to the lungs which is a problem widely encountered during inhalation of dry powder formulations. In the prior art, various methods and devices are developed in order to deal with this problem.

It is found that the most suitable method delivering effective amount of dose to target area which is lung of the patient and for making patient bring about inhalation in a most suitable way is to inhale said medicament composition as dry powder form and to inhale said medicament from a peelable blister strip. It is found that compared to the other methods for delivery of dry powder formulation which comprise inhalation of dry powder formulation containing (A), (S), and (B) in effective amounts from capsule or from reservoir or from separate blister strips each of which contains a kind of active agent, the method for delivering said dry powder formulation from a peelable blister strip minimize the drawbacks caused by adhesion force of micronized dry powder and the amount of medicament which reaches lungs increases.

Further disclosed is a method comprising delivery or medicament composition containing (A), (S) and (B) in effective amounts and optionally a pharmaceutically acceptable carrier from a blister strip by using dry powder inhaler for the treatment of respiratory diseases especially allergic disease.

Tiotropium (A) may be in the form of a pharmaceutically acceptable salt, hydrate, solvate, ester, polymorph, free base, crystal and/or amorphous form thereof in the invention tiotropium bromide is used. All of crystal and amorphous forms of tiotropium, which shows different polymorphic forms, can be used.

Also, tiotropium used within the scope of invention can be in the form of anhydrate and hydrate, preferably, tiotropium bromide anhydrate is used.

In an embodiment of the invention, corticosteroid can be selected from preferably ciclesonide (S) or its pharmaceutically acceptable salts, hydrates, solvates, esters, polymorphs, enantiomers, rasemates, free base, crystal and amorph forms.

In an embodiment of the invention, salt of cromoglicic acid and/or nedocromil (B) can be selected from pharmaceutically acceptable salts thereof. Preferably, it can be either sodium cromoglicate and/or nedocromil sodium.

Dry powder formulation in accordance with the present invention administered via inhalation route optionally contains pharmaceutically acceptable carrier. The carrier can be selected from a group comprising arabinose, glucose, fructose, ribose, mannose, sucrose, trehalose, lactose, maltose, starches, dextran, sugar alcohols such as mannitol and saccharides.

The medicament composition in accordance with the present invention is in the form of micronized dry powder particles. The active agents present in said medicament composition have average particle size in the range of 1 to 20 µm, preferably in the range of 1 to 6 µm. The carrier present in said medicament composition typically has average particle size of not more than 300 µm, preferably not more than 210 µm. Salt of cromoglicic acid and/or nedocromil present in said medicament composition both as an active agent and as an excipient, that has average particle size in the range of 1 to 20 µm and in the range of 10 to 300 µm.

The cavity volume of each blister in the peelable blister strip contained in the dry powder inhaler which is used for delivery of said medicament composition to the lung, is in the range of 20 to 30 mm³, preferably in the range of 21 to 25 mm³, most preferably in the range of 22 to 23 mm³.

A lid sheet and a base sheet which constitutes said blister strip are closed very tightly to provide impermeability by using suitable method. The lid sheet or the base sheet of the peelable blister strip consists of three layers. Two of these layers are polymeric layers and the other one is aluminium foil. Aliminium foil is used both in the lid sheet and in the base sheet of the peelable blister strip to provide high humidity and gas protection because of that aluminium foil is conventionally used in both the lid sheet and the base sheet of the peelable blister strip for high humidty and gas protection. These layers must have the sufficient thickness which provides the protection for the stability of humidity sensitive dry powder formulation which is stored in blister cavity. Because of this reason, the thickness of aluminium foil that is used in the lid sheet and the base sheet of the peelable blister strip is in the range of 10 to 40 µm, preferably of 15 to 30 µm.

Two of the layers contained by the lid sheet and the base sheet of the peelable blister strip are polymeric layers. These polymeric layers may be made from either same or different polymers. The thickness of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of polymer used.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing.

The polymers used for forming polymeric layers are preferably selected from a group comprising thermo-plastic polymers such as polyethylene, polypropylene, polystyrene, polyolefin, polyamide, polyvinyl chloride, polyurethane.

The layers which are used for making up the lid sheet and the base sheet of said blister strip are preferably same for each sheet; however the polymeric substances used for forming polymeric layers are preferably different from each other. Moreover, each of the blister cavities which constitute the peelable blister strip can be different in shape as long as it has the properties defined above.

Devices used to inhale the dry powder formulation in accordance with the present invention may be multi dose inhalers present in the prior art. For this reason, the invention provides a medicament composition as mentioned before. Also disclosed is a method for delivery of medicament composition to patient's lungs effectively as mentioned before.

The medicament composition in accordance with the present invention containing active agents which is preferably in dry powder form is stored in the peelable blister strip and during inhalation 2 to 50 milligram of said medicament composition including one for each dose of A,S and B is delivered to patients by using a multi dose inhaler, after each movement of the device.

In the medicament composition of present invention (A) is present in the amount of 1 to 50 µg, preferably 1 to 30 µg, (S) is present in the amount of 5 to 750 µg, preferably 5 to 550 µg, (B) is present in the amount of 1 to 50 mg, preferably 1 to 25 mg. Accordingly, the ratio of the total weight of (A) and (S) to the weight of (B) in said medicament composition is in the range of 1:5 to 1:1500. Additionally, in medicament composition pharmaceutically acceptable carrier can be used for the purpose of adjusting each dose of medicament composition, which is in dry powder form, to the range of 2 to 50 mg.

Medicament composition in accordance with present invention can be used for the treatment of many respiratory diseases such as asthma, chronic obstructive pulmonary disorder (COPD) and allergic rhinitis. Accordingly, the respiratory diseases include but are not restricted to; allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition in accordance with the present invention is used especially for the symptomatic treatment of asthma, allergic rhinitis and COPD.

This present invention, is explained with examples given below, but it is not restricted to these examples. Parts given in examples represent the weights of ingredients.

### Example 1

The medicament composition in dry powder form which is stored in the peelable blister strip to be delivered by using multi-dose inhaler and is prepared by mixing 18 parts of tiotropium bromide anhydrate, 200 parts of ciclesonide and 8000 parts of sodium cromoglicate, having average particle size of 1 to 5 µm, and 10000 parts of sodium cromoglicate having average particle size of not more than 300 µm, which are micronized in an air jet mill, and 5000 parts of carrier which is suitable as a pharmaceutical such as lactose.

As given in the following example, tiotropium bromide anhydrate may be extended tiotropium or pharmaceutically acceptable salts and polymorphs (A) thereof; ciclesonide may be extended pharmaceutically acceptable salts, enantiomers, racemates, polymorphs, solvates, hydrates, amorph and/or crystal forms (S) thereof, sodium cromoglicate (B) may be extended salts of cromoglicic acid and pharmaceutically acceptable salts of nedocromil, more or less amount of pharmaceutically acceptable carrier may optionally be added, and, thus example 1 is repeated as following:

| | Amount of Tiotropium Bromide | Amount of Ciclesonide | Amount of A salt of Cromoglicic acid |
|---|---|---|---|
| | parts | parts | parts |
| Example 2 | 9 | 100 | 12000 |
| Example 3 | 9 | 100 | 20000 |
| Example 4 | 9 | 100 | 30000 |
| Example 5 | 9 | 100 | 10000 |
| Example 6 | 12 | 200 | 12000 |
| Example 7 | 12 | 200 | 20000 |
| Example 8 | 12 | 400 | 30000 |
| Example 9 | 12 | 400 | 10000 |
| Example 10 | 15 | 400 | 12000 |
| Example 11 | 15 | 400 | 20000 |
| Example 12 | 15 | 400 | 30000 |
| Example 13 | 15 | 400 | 10000 |
| Example 14 | 18 | 200 | 12000 |
| Example 15 | 18 | 200 | 20000 |
| Example 16 | 18 | 400 | 30000 |
| Example 17 | 18 | 400 | 10000 |
| Example 18 | 21 | 500 | 12000 |
| Example 19 | 21 | 500 | 20000 |
| Example 20 | 21 | 500 | 30000 |
| Example 21 | 21 | 500 | 10000 |
| Example 22 | 24 | 600 | 12000 |
| Example 23 | 24 | 600 | 20000 |
| Example 24 | 24 | 600 | 30000 |
| Example 25 | 24 | 750 | 10000 |
| Example 26 | 27 | 400 | 18000 |
| Example 27 | 27 | 400 | 20000 |
| Example 28 | 27 | 400 | 30000 |
| Example 29 | 27 | 400 | 15000 |
| Example 30 | 30 | 200 | 17000 |
| Example 31 | 30 | 200 | 20000 |
| Example 32 | 30 | 600 | 30000 |
| Example 33 | 30 | 600 | 50000 |

## Claims

1. A medicament composition containing combination of tiotropium bromide (A) and ciclesonide or a pharmaceutically acceptable salt, hydrate, solvate, ester, polymorph, enantiomer, rasemate, free base, crystal and amorph forms thereof (S) for use in the treatment of respiratory disorder by inhalation route **characterized in that** said medicament composition contains of a pharmaceutically acceptable salt of cromoglicic acid and/or nedocromil (B) in an effective amount.

2. A medicament composition according to claim 1, wherein tiotropium bromide (A) is tiotropium bromide anhydrate.

3. A medicament composition according to claim 1, wherein ciclesonide (S) is a pure enantiomer, mixture of enantiomers and/or racemate.

4. A medicament composition according to any one of the preceding claims, wherein salt of cromoglicic acid (B) is sodium cromoglicate.

5. A medicament composition according to any one of claims 1 to 3, wherein salt of nedocromil (B) is nedocromil sodium.

6. A medicament composition according to claim 1, wherein said medicament composition used locally for the treatment of respiratory diseases by inhalation route contains micronized dry powder.

7. A medicament composition in dry powder form according to claim 6, wherein said medicament composition is administered directly to airways by using a dry powder inhalation device.

8. A dry powder medicament composition according to claim 6, wherein active agent has an average particle size in the range of 1 to 20 µm, preferably in the range of 1 to 6 µm.

9. A medicament composition in dry powder form according to any one of the preceding claims, wherein tiotropium bromide (A) is present in the amount of 1 to 50 µg, ciclesonide (S) is present in the amount of 5 to 750 µg and a salt of cromoglicic acid and/or nedocromil (B) is present in the amount of 1 to 50 mg for each dose and any amount of pharmaceutically suitable carrier sufficient for adjusting each dose to an amount of 2-50 milligrams in total.

10. A medicament composition in dry powder form according to claim 9, wherein the ratio of the total weight of tiotropium bromide (A) and ciclesonide (S) to weight of a salt of cromoglicic acid (B) is in the range of 1:5 to 1:1500.

11. A medicament composition in dry powder form according to claim 6, wherein said medicament optionally contains a pharmaceutically acceptable carrier.

12. A medicament composition according to claim 11 wherein said pharmaceutically acceptable carrier is selected from a group comprising monosaccharide, disaccharide, polysaccharide and oligosaccharide.

13. Use of tiotropium bromide (A), ciclesonide (S) and a salt of cromoglicic acid and/or nedocromil (B) for the reparation of a medicament according to any one of preceding claims for use in the treatment of inflammatory or obstructive respiratory diseases.

## Patentansprüche

1. Eine Medikamentenzusammensetzung , die zur Verwendung in der Behandlung von Atemstörungen durch den Inhalationsweg die Kombination von Tiotropiumbromid (A) und Ciclesonid oder einem pharmazeutischen annehmbaren Salz, Hydrat, Solvat, Ester, Polymorph, Enantiomer, rasemate, freie Base, Kristall und amorphen Formen (S) beinhaltet; **dadurch gekennzeichnet, dass** die besagten Medikamentenzusammensetzung ein pharmazeutisch annehmbares Salz von Cromoglicinsäure und / oder Nedokromilen (B) in einer wirksamen Menge beinhaltet.

2. Eine Medikamentenzusammensetzung gemäß Anspruch 1, wobei Tiotropiumbromid (A) Tiotropiumbromid Anhydrat ist.

3. Eine Medikamentenzusammensetzung gemäß Anspruch 1, wobei Ciclesonid (S) ein Gemisch vom reinen Enantiomeren und/oder von Enantiomeren und Racemate ist

4. Eine Medikamentenzusammensetzung gemäß einem der vorhergehenden Ansprüche, wobei Salz von Cromoglicinsäure (B) Natriumcromoglicat ist.

5. Eine Medikamentenzusammensetzung gemäß einem der Ansprüche von 1 bis 3, wobei Salz von Nedokromil (B) Nedocromilnatrium ist.

6. Eine Medikamentenzusammensetzung gemäß Anspruch 1, wobei die besagten Medikamentenzusammensetzung, die lokal für die Behandlung von Atemwegserkrankungen durch den Inhalationsweg verwendet wird, das mikronisierte Pulver enthält.

7. Eine Medikamentenzusammensetzung in trockener Pulverform nach Anspruch 6, wobei die besagten Medikamentenzusammensetzung direkt den Atemwegen durch Verwendung eines Trockenpulver-Inhalationsgeräts verabreicht.

8. Eine Trockenpulver-Medikamentenzusammensetzung nach Anspruch 6, wobei der Wirkstoff eine durchschnittliche Teilchengröße im Bereich von 1 bis 20 µm, vorzugsweise im Bereich von 1 bis 6 µm hat.

9. Eine Medikamentenzusammensetzung in trockener Pulverform gemäß einem der vorhergehenden Ansprüche, wobei Tiotropiumbromid (A) in der Menge von 1 bis 50 µm vorhanden ist; Ciclesonid (S) in der Menge von 5 bis 750 µg vorhanden ist; und ein Salz von Cromoglicinsäure und / oder Nedokromil (B) in der Menge von 1 bis 50 mg für jede Dosis vorhanden ist und die Zusammensetzung einen pharmazeutisch geeigneten Träger enthält, der ausreichend zur Einstellung jeder Dosis insgesamt einer Menge von 2-50 mg ist.

10. Eine Medikamentenzusammensetzung in trockener Pulverform nach Anspruch 9, wobei das Verhältnis des Gesamtgewichts von Tiotropiumbromid (A) und Ciclesonide (S) zu einem Salz von Cromoglicinsäure (B) im Bereich von 1: 5 bis 1: 1500 liegt.

11. Eine Medikamentenzusammensetzung in trockener Pulverform nach Anspruch 6, wobei das besagte Medikament gegebenenfalls einen pharmazeutisch annehmbaren Träger enthält.

12. Eine Medikamentenzusammensetzung gemäß Anspruch 11, wobei der pharmazeutisch annehmbare Träger aus einer Gruppe gewählt wird, die Monosaccharid, Disaccharid, Polysaccharid und Oligosaccharid enthält.

13. Verwendung von Tiotropiumbromid (A), Ciclesonid (S) und einem Salz von Cromoglicinsäure und / oder Nedokromil (B) zur Herstellung eines Medikamentes gemäß einem der vorhergehenden Ansprüche zur Verwendung in der Behandlung von entzündlichen oder obstruktiven Atemwegserkrankungen.

## Revendications

1. Une composition de médicament contenant une combinaison du bromure de tiotropium (A) et du ciclésonide ou un sel, un hydrate, un solvat, un ester, un polymorphe, un énantiomère, un racémate, une base libre, un cristal et des formes amorphes, pharmaceutiquement acceptables, de celui-ci (S) afin d'utiliser, par inhalation, dans le traitement des troubles respiratoires, **caractérisée en ce que** ladite composition de médicament contient un sel pharmaceutiquement acceptable de l'acide cromoglicique et/ou nédocromil (B) dans une quantité efficace.

2. Une composition de médicament selon la revendication 1, dans laquelle le bromure de tiotropium (A) est le bromure de tiotropium anhydre.

3. Une composition de médicament selon la revendication 1, dans laquelle le ciclésonide (S) est un énantiomère pur, un mélange d'énantiomères et/ou racémate.

4. Une composition de médicament selon l'une quelconque des revendications précédentes, dans laquelle le sel d'acide cromoglicique (B) est le cromoglicate de sodium.

5. Une composition de médicament selon l'une quelconque des revendications 1 à 3, dans laquelle le sel de nédocromil (B) est le nédocromil de sodium.

6. Une composition de médicament selon la revendication 1, dans laquelle ladite composition de médicament qui est utilisé localement pour le traitement, par inhalation, des troubles respiratoires contient une poudre micronisée sèche.

7. Une composition de médicament sous la forme d'une poudre sèche selon la revendication 6, dans laquelle ladite composition de médicament est administrée directement dans les voies respiratoires en utilisant un dispositif d'inhalation de poudre sèche.

8. Une composition de médicament sous la forme d'une poudre sèche selon la revendication 6, dans laquelle le principe actif a une taille particulaire moyenne comprise dans la plage de 1 à 20 µm, de préférence dans la plage de 1 à 6 µm.

9. Une composition de médicament sous la forme d'une poudre sèche selon l'une quelconque des revendications précédentes, dans laquelle le bromure de tiotropium (A) est présent dans la plage de 1 à 50 µm, le ciclésonide (S) est présent dans la plage de 5 à 750 µm et un sel d'acide cromoglicique et/ou nédocromile (B) est présent dans la plage de 1 à 50 mg pour chaque dose et un porteur pharmaceutiquement approprié en quelque quantité suffisant pour ajuster chaque dose en quantité totale de 2-50 milligrammes.

10. Une composition de médicament sous la forme d'une poudre sèche selon la revendication 9, dans laquelle le rapport entre le poids total du bromure de tiotropium (A) et le ciclésonide (S) et le poids d'un sel d'acide cromologlicique est dans la plage de 1:5 à 1:1500.

11. Une composition de médicament sous la forme d'une poudre sèche selon la revendication 6, dans laquelle ledit médicament contient éventuellement un porteur pharmaceutiquement acceptable.

12. Une composition de médicament selon la revendication 11, dans laquelle ledit porteur pharmaceutiquement acceptable est choisi dans un group comprenant des monosaccharides, des disaccharides, des polysaccharides et des oligosaccharides.

13. L'utilisation de bromure de tiotropium (A), ciclésonide (S) et un sel d'acide cromoglicique et/ou nédocromil (B) pour la préparation d'un médicament selon l'une quelconque des revendications précédentes afin d'utiliser dans le traitement des maladies inflammatoires ou respiratoires obstructives.
